**Europäisches Patentamt**

**European Patent Office**

**Office européen des brevets**

(19)

(11) Veröffentlichungsnummer : **0 059 792
B1**

(12) **EUROPÄISCHE PATENTSCHRIFT**

(45) Veröffentlichungstag der Patentschrift :
25.07.84

(51) Int. Cl.³ : **C 07 C102/00**, C 07 C103/127,
C 07 C103/34

(21) Anmeldenummer : 81109071.1

(22) Anmeldetag : 28.10.81

(54) Verfahren zur Stabilisierung wässeriger Lösungen von Acetoacetamiden.

(30) Priorität : 06.03.81 DE 3108622

(43) Veröffentlichungstag der Anmeldung :
15.09.82 Patentblatt 82/37

(45) Bekanntmachung des Hinweises auf die Patenterteilung : 25.07.84 Patentblatt 84/30

(84) Benannte Vertragsstaaten :
BE CH DE FR GB IT LI

(56) Entgegenhaltungen :
DE-C- 1 125 418
R.O.C. NORMAN "Principles of Organic Synthesis", 2.
Auflage, 1978 Chapman and Hall Ltd., London pages
686 bis 687

(73) Patentinhaber : WACKER-CHEMIE GMBH
Prinzregentenstrasse 22
D-8000 München 22 (DE)

(72) Erfinder : Thyret, Helmut, Dr. Dipl.-Chem.
Marienberger Strasse 76
D-8263 Burghausen (DE)

**Beschreibung**

Acetoacetamide, worunter insbesondere Acetoacetamid, N-Methyl-acetoacetamid und N,N-Dimethyl-acetoacetamid zu verstehen sind, finden als Zwischenprodukte für die Herstellung von Arzneimitteln, Pflanzenschutzmitteln und Farbstoffen Verwendung, wobei sie aufgrund der leichteren Handhabbarkeit vorteilhaft in Form wässeriger Lösungen eingesetzt werden.

Die Herstellung verdünnter wässeriger Lösungen von Acetoacetamiden durch Umsetzung von Diketen mit überschüssigem Ammoniak oder wasserlöslichen, aliphatischen primären oder sekundären Aminen in Wasser ist bekannt (vgl. « Methoden der organischen Chemie » von Houben-Weyl, Bd. 7/4, 4. Auflage 1968, Seite 233-236 und US-PS 2 152 132).

Es ist ferner bekannt, konzentrierte wässerige Lösungen von Acetoacetamiden durch Umsetzung von Diketen mit Salzen aus schwachen Säuren und Ammoniak, wie Ammoniumcarbonat oder Ammoniumbicarbonat oder Salzen aus einer Carbonsäure und primären aliphatischen Aminen in annähernd stöchiometrischem Verhältnis in wässerigem Medium herzustellen (vgl. CH-PS 287 558 und US-PS 2 615 917). Da hierbei während der Umsetzung eine $CO_2$-Entwicklung stattfindet, können diese Verfahren nicht kontinuierlich unter ständiger Kontrolle des pH-Wertes durchgeführt werden, von der die Bildung von Nebenprodukten abhängig ist. Deshalb wird insbesondere bei der Herstellung von konzentrierten wässerigen Acetoacetamidlösungen durch Umsetzung von Diketen und Ammoniak mit einem Ammoniaküberschuß gearbeitet, damit während der Umsetzung ein pH-Wert von 9,5 bis 10 aufrecht erhalten bleibt (vgl. DE-AS 1 125 418, die der GB-PS 828 423 entspricht). Desgleichen erfolgt die Konzentrierung verdünnter wässeriger Acetoacetamidlösungen in Gegenwart von überschüssigem Ammoniak, dem offensichtlich eine zersetzungsverhindernde bzw. stabilisierende Wirkung auf das Acetoacetamid in der wässerigen Lösung zugeschrieben wird (vgl. GB-PS 832 956).

In R.O.C. Norman « Principles of Organic Synthesis », 2. Auflage, 1978, Chapman and Hall Ltd., London, S. 686 bis 687 wird die Selbstkondensation von Acetessigester beschrieben, die durch Einwirkung von basischen Kondensationsmitteln unter drastischen Bedingungen (200 °C) zur Bildung von Dehydracetsäure führt, während durch Behandlung mit starken Säuren (konz. Schwefelsäure oder Chlorwasserstoff) ein Gemisch von Isodehydracetsäure und ihrem Ethylester entsteht. Aus der bekannten Tatsache, daß unter den genannten drastischen Bedingungen bei Acetessigestern eine Selbstkondensation erzwungen werden kann, können jedoch keine Rückschlüsse auf das mögliche Verhalten von Acetoacetamiden in wässeriger Lösung gezogen werden, die als β-Ketocarbonsäureamide mit den β-Ketocarbonsäureestern hinsichtlich ihrer Reaktionsfähigkeit nicht direkt vergleichbar sind.

Der Stand der Technik vermittelt demnach die Lehre, daß wässerige Acetoacetamidlösungen, die in Gegenwart von überschüssigem Ammoniak bzw. Amin hergestellt worden sind, weniger Nebenprodukte enthalten und demgemäß stabiler sein sollten, als solche, die unter praktisch neutralen Bedingungen erhalten worden sind.

Es wurde jedoch festgestellt, daß auch derartige wässerige Lösungen mit einem pH-Wert von > 9 nur begrenzt lagerfähig sind und zwar aufgrund der Zersetzungsneigung der Acetoacetamide selbst, die durch die angenommene Stabilisierungswirkung von Ammoniak nicht in ausreichendem Maße kompensiert werden kann.

Die Lagerbeständigkeit von wässerigen Acetoacetamidlösungen ist dabei sowohl von der Konzentration, das heißt dem Gehalt der Acetoacetamide und der Lagertemperatur, als auch von der Art der Acetoacetamide selbst abhängig. Hierunter ist zu verstehen, daß die Stabilität in der Reihenfolge der unsubstituierten über die monoalkylsubstituierten bis zu den dialkylsubstituierten Acetoacetamiden zunimmt.

Der Erfindung liegt daher die Aufgabe zugrunde, ein Verfahren zur Stabilisierung wässeriger Lösungen von Acetoacetamiden zur Verfügung zu stellen, die durch Umsetzung von Diketen mit überschüssigem Ammoniak oder wasserlöslichen, aliphatischen primären oder sekundären Aminen in wässeriger Lösung unter Aufrechterhaltung eines pH-Wertes von 8 bis 10 hergestellt worden sind.

Diese Aufgabe wird erfindungsgemäß dadurch gelöst, daß die wässerige Lösung nach beendeter Umsetzung durch Zugabe einer Säure auf einen pH-Wert von 6 bis 7,5 eingestellt wird. Als Säuren sind organische Carbonsäuren, die außer der Carboxylgruppe keine funktionellen Gruppen aufweisen, wie Essigsäure bevorzugt. Es können jedoch auch verdünnte anorganische Säuren, wie Schwefelsäure oder Phosphorsäure verwendet werden.

Die so hergestellten wässerigen Lösungen mit schwach saurer bis neutraler Reaktion zeigen auch nach einer Standzeit bis zu etwa 100 Tagen bei Raumtemperatur praktisch keine Abnahme des ursprünglich vorhandenen Acetoacetamidgehalts. Bei höherer Temperatur, das heißt bis zu etwa 80 °C ist die relative Abnahme des Acetoacetamidgehalts der ursprünglichen Lösung immer noch beträchtlich niedriger als derjenige einer basischen Lösung mit gleichem Ausgangsgehalt, wie durch Vergleichsversuche bestätigt werden konnte.

In den folgenden Beispielen wurde der Gehalt der entsprechenden Acetoacetamide, die jeweils in bekannter Weise durch Umsetzung von Diketen mit überschüssigem Ammoniak bzw. Methylamin oder Dimethylamin in wässeriger Lösung hergestellt worden waren, zu Beginn und nach der angegebenen Standzeit bei der angegebenen Temperatur durch gaschromatographische Analyse ermittelt.

Beispiel 1

Stabilisierung von einer 30 Gew.-%-igen wässerigen Lösung von Acetoacetamid durch Erniedrigen des pH-Wertes mittels Essigsäure, Änderung des Gehalts bei Raumtemperatur (ca. 20 °C).

| Standzeit (Tage) | A Gehalt (Gew.-%) | B Gehalt (Gew.-%) | Standzeit (Tage) | C Gehalt (Gew.-%) |
|---|---|---|---|---|
| 0 | 29,49 | 29,16 | 0 | 30,53 |
| 30 | 22,61 | 28,28 | 29 | 31,55 |
| 56 | 20,75 | 25,07 | 58 | 29,95 |
| 100 | 20,06 | 25,02 | 87 | 30,36 |

A : Ausgangs-pH-Wert 9,2 ohne Zugabe von Säure
B : Ausgangs-pH-Wert 9,2 mit Essigsäure auf pH 7,5 eingestellt
C : Ausgangs-pH-Wert 9,2 mit Essigsäure auf pH 7,0 eingestellt.

Beispiel 2

Stabilisierung einer 70 Gew.-%-igen wässerigen Lösung von N-Methylacetoacetamid durch Erniedrigen des pH-Wertes mittels Essigsäure, Änderung des Gehalts bei 80 °C.

| Standzeit (Tage) | D Gehalt (Gew.-%) | E Gehalt (Gew.-%) |
|---|---|---|
| 0 | 70,0 | 70,0 |
| 15 | 50,0 | 54,4 |
| 28 | 30,0 | 35,5 |
| 33 | 26,2 | 34,0 |
| relative Abnahme nach 33 Tagen | 62,6 % | 51,4 % |

D : Ausgangs-pH-Wert 7,8, ohne Zugabe von Säure
E : Ausgangs-pH-Wert 7,8, eingestellt auf 7,0 mit Essigsäure.

Beispiel 3

Stabilisierung einer 70 Gew.-%-igen wässerigen Lösung von N,N-Dimethylacetoacetamid durch Erniedrigen des pH-Wertes mittels Essigsäure, Änderung des Gehalts bei 80 °C.

| Standzeit (Tage) | F Gehalt (Gew.-%) | G Gehalt (Gew.-%) | H Gehalt (Gew.-%) | I Gehalt (Gew.-%) |
|---|---|---|---|---|
| 0 | 69,2 | 69,6 | 66,0 | 63,7 |
| 14 | 59,0 | 59,1 | 57,7 | 57,0 |
| relative Abnahme nach 14 Tagen | 14,7 % | 15,1 % | 12,6 % | 10,5 % |

F : Ausgangs-pH-Wert 8,2, ohne Zugabe von Säure
G : Ausgangs-pH-Wert 7,8, ohne Zugabe von Säure
H : Ausgangs-pH-Wert 8,2, mit Essigsäure auf pH 7,0 eingestellt
I : Ausgangs-pH-Wert 8,2, mit Essigsäure auf pH 6,2 eingestellt.

**Ansprüche**

1. Verfahren zur Stabilisierung wässeriger Lösungen von Acetoacetamiden, die durch Umsetzung von Diketen mit überschüssigem Ammoniak oder wasserlöslichen aliphatischen, primären oder sekundären Aminen in wässeriger Lösung unter Aufrechterhaltung eines pH-Wertes von 8-10 hergestellt worden sind, dadurch gekennzeichnet, daß die wässerige Lösung nach beendeter Umsetzung durch Zugabe einer Säure auf einen pH-Wert von 6 bis 7,5 eingestellt wird.

2. Verfahren nach Anspruch 1, dadurch gekennzeichnet, daß als Säure Essigsäure verwendet wird.

**0 059 792**

Claims

1. Process for stabilising aqueous solutions of acetoacetamides that have been manufactured by reacting diketene with excess ammonia or with watersoluble, aliphatic, primary or secondary amines in aqueous solution while maintaining a pH of from 8 to 10, characterised in that, after the reaction has finished, the aqueous solution is adjusted to a pH of from 6 to 7.5 by the addition of an acid.

2. Process according to claim 1, characterised in that acetic acid is used as the acid.

**Revendications**

1. Procédé pour stabiliser des solutions aqueuses d'acétoacétamides que l'on a préparées par réaction, en solution aqueuse, du dicétène avec un excès d'ammoniac ou d'amines aliphatiques primaires ou secondaires, solubles dans l'eau, tout en maintenant le pH entre 8 et 10, procédé caractérisé en ce qu'on règle le pH de la solution aqueuse, après la fin de la réaction, à une valeur comprise entre 6 et 7,5 par addition d'un acide.

2. Procédé selon la revendication 1 caractérisé en ce qu'on utilise l'acide acétique comme acide.

4